# EUROPEAN PATENT APPLICATION

(11) **EP 1 820 789 A1**
(43) Date of publication of application: **22.08.2007**
(21) Application number: 06101566.5
(22) Date of filing: 13.02.2006
(51) Int. Cl.: C07C 29/149, C07C 31/38

(54) **Process for the manufacture of fluorinated alcohols**

(71) Applicant: SOLVAY (Société Anonyme), 1050 Bruxelles (BE)
(72) Inventor: Buyle, Olivier, 1367, AUTRE-EGLISE (BE); Lorent, Karol, 6182, SOUVRET (BE); Mathieu, Véronique, 1300, WAVRE (BE)
(74) Representative: Mross, Stefan P.M.

(57) **Abstract**

Process for the manufacture of fluorinated alcohols having the formula

CₙFₘH₂ₙ₊₁₋ₘCH₂OH (1)

wherein n is 1 or 2; and m is an integer from 1 to 5, but not larger than 2n+1; by hydrogenating the corresponding fluorinated carboxylic acids and/or their derivates having the formula

CₙFₘH₂ₙ₊₁₋ₘCOR (2)

wherein n and m have the above meanings and R represents OH, Cl, Br, F, and OR', wherein R' is a hydrocarbon rest, in the presence of a catalyst and of water but excluding the hydrogenation of trifluoroacetic acid to form trifluoroethanol.

## Description

The present invention relates to a process for the manufacture of fluorinated alcohols and in particular to a process for the manufacture of fluorinated alcohols by hydrogenating fluorinated carboxylic acids, their acid halides or their esters with hydrogen in the presence of a catalyst.

Fluorinated alcohols are very attractive chemical compounds that find wide use. For example, pentafluoropropanol (PFP) is used for various applications including the use as a solvent and the use as an intermediate for the manufacture of esters, ethers, carbonates, etc.

US 4,273,947 discloses the heterogeneous hydrogenation of fluorine-containing alkyl, cycloalkyl, and benzene carboxylic acids to the corresponding primary alcohols in the presence of hydrogen gas. The hydrogenation can be carried out in the liquid or vapour phase in the presence of a solid rhodium or iridium catalyst, employed as the metal, metallic oxide, or mixture thereof. A preferred embodiment is the hydrogenation of trifluoroacetic acid in the liquid phase to 2,2,2,-trifluoroethanol. Perfluoropropionic acid is mentioned among many other fluorinated carboxylic acids which can be used in the process of that patent.

US 4,273,947 teaches that the initial presence of an aqueous system may be deleterious to catalytic activity.

US 4,533,711 discloses the catalytic hydrogenation of trifluoroacetic acid in the absence or the presence of water.

US 3,663,629 discloses a process for the preparation of a compound of the formula CₙF₂ₙ₊₁-CH₂OH in which n is a number equal to or greater than 3, preferably 3 to 12, by hydrogenation of a compound of the formula CₙF₂ₙ₊₁₋CO₂R in which R is hydrogen or an alkyl group of preferably 1 to 4 carbon atoms and n is as defined above, at elevated pressure and with a ruthenium catalyst, wherein the hydrogenation is carried out in the presence of 4 to 100 % by weight, based on CₙF₂ₙ₊₁-CO₂R, of water at a temperature of about 80 to 240°C and at a pressure of about 5 to 700 kp./cm². In a preferred embodiment, the ruthenium catalyst contains about 5 wt% of ruthenium supported on carbon.

An object underlying the present invention was therefore to provide an efficient process for the manufacture of fluorinated alcohols by hydrogenation of fluorinated carboxylic acids and/or their derivates which shows a high yield, notably under relatively mild conditions of temperature and/or pressure.

This object is achieved by a process for the manufacture of fluorinated alcohols having the formula

CₙFₘH₂ₙ₊₁₋ₘCH₂OH (1)

wherein n is 1 or 2; and m is an integer from 1 to 5, but not larger than 2n+1; by hydrogenating the corresponding fluorinated carboxylic acids and/or their derivates having the formula

CₙFₘH₂ₙ₊₁₋ₘCOR (2)

wherein n and m have the above meanings and R represents OH, Cl, Br, F, and OR', wherein R' is a hydrocarbon rest, preferably a C₁-C₅ alkyl, in the presence of a catalyst and of water, but excluding the hydrogenation of trifluoroacetic acid to form trifluoroethanol.

The process according to the present invention is especially suitable for the production of fluorinated alcohols of formula 1 which are selected from the group consisting of pentafluoropropanol (CF₃CF₂CH₂OH), trifluoropropanol (CF₃CH₂CH₂OH), and difluoroethanol (CF₂HCH₂OH).

These fluorinated alcohols are preferably produced according to the present invention by hydrogenating the corresponding compounds of formula 2 which are preferably selected from the group consisting of pentafluoropropionic acid (CF₃CF₂COOH), trifluoropropionic acid (CF₃CH₂COOH) and difluoroacetic acid (CF₂HCOOH).

More preferably, difluoroethanol is produced by hydrogenation of the corresponding fluorinated esters. Preferred fluorinated esters according to formula (2) are the methyl and ethyl difluoroacetate esters, preferably the methyl difluoroacetate ester.

Most advantageously, pentafluoropropanol (CF₃CF₂CH₂OH) is produced by hydrogenating pentafluoropropionic acid (CF₃CF₂COOH) in the process according to the present invention.

The pentafluoropropionic acid to be used can be obtained via various processes. Preferably, pentafluoropropionic acid is produced by a method comprising the step of reacting CF₂CF₂ with CO₂ and a source of fluoride ions in the presence of diethylene glycol dimethyl ether (diglyme). The source of fluoride ions is preferably an alkali salt of hydrofluoric acid. Most preferred is the use of CsF.

The concentration of water in the process according to the present invention is preferably from 10 to 70 % by weight, more preferably from 30 to 50 % by weight, most preferably from 35 to 45 % by weight, based upon the total weight of fluorinated carboxylic acids and/or their derivatives, water and catalyst.

The hydrogenation of the fluorinated carboxylic acids and/or their derivates having the formula (2) in the process of the invention is preferably carried out under elevated hydrogen pressure, i.e. above atmospheric pressure. The hydrogen pressure is preferably from 1.5 to 70 bar.

The process is moreover preferably carried out at a temperature from 70 to 140°C.

The reaction time can vary broadly. In general, the reaction time is however from 1 to 30 hours.

The catalyst in the process according to the present invention is not particularly limited. Preferred catalysts comprise Ir, Rh or Ru on carbon (Ir/C, Rh/C and Ru/C, respectively). Particularly preferred are Rh/C and Ru/C catalysts. Most preferred is a Rh/C catalyst.

In these catalysts, the Ir, Rh or Ru is used preferably in an amount of from 2 to 10 % by weight, more preferably, 3 to 7 % by weight, with respect to the weight of the carbon support.

The catalyst is preferably used in an amount of 0.5 to 7 % by weight, more preferably, 1 to 3.5 % by weight, based upon the total weight of fluorinated carboxylic acids and/or their derivatives, water and catalyst (i.e. = g catalyst/(g catalyst + g acid etc. + g water) x 100).

It was found moreover that, in general, fluorinated carboxylic acids of the formula

CₙFₘH₂ₙ₊₁₋ₘCOOH (3)

wherein n is an integer from 3 to 10; and m is an integer from 1 to 2n+1, can be manufactured by reacting an ethylenically unsaturated, at least partially fluorinated compound of the formula

Cₙ₋₁FₘH₂ₙ₋₂₋ₘ (4)

with CO₂ and a source or fluoride ions in the presence of diethylene glycol dimethyl ether (diglyme). The source of fluoride ions is preferably an alkali salt of hydrofluoric acid.

The fluorinated alcohol of formula 1 obtained according to the process of the present invention can be separated from the reaction mixture for example by means of distillation, filtration or decanting.

If the fluorinated alcohol of formula 1 to be obtained has an F/H atomic ratio greater than or equal to 1, the fluorinated alcohol in particular pentafluoropropanol is preferably separated from the reaction mixture by means of filtration or decanting.

If the fluorinated alcohol of formula 1 to be obtained is soluble in water, a solvent will be preferably added during or after the hydrogenation reaction which is essentially non-miscible with water, but wherein the fluorinated alcohol is soluble. This allows the extraction of the desired fluorinated alcohol by means of the added solvent.

Finally, the obtained fluorinated alcohol is in general dried over concentrated sulphuric acid or molecular sieves up to a residual water content of about 50 ppm.

The process of the present invention allows to obtain an increased yield of fluorinated alcohols, in particular pentafluoropropanol, and of fluorinated acid, in particular pentafluoropropionic acid, while it is at the same time possible to avoid drastic reaction conditions (high temperatures, high pressures, long reaction times) and cumbersome separation steps which in general would lead to low yields after purification.

In the following, the invention will be described in more detail by examples.

### Examples

### Synthesis of Pentafluoropropanol

2.15 g of 5 wt% Rh/C catalyst was introduced together with 32.8 g of water and 39.8 g of perfluoropropionic acid at room temperature into an autoclave reactor. The reactor was then purged with helium. H₂ was bubbled into the solution via a dipping tube at a flow-rate of 8 liter / h. The pressure was set to 10 bar and the reactor temperature was increased to 90°C. After 18 hours, the autoclave was cooled down to room temperature and vented. The collected liquid phase was filtered and then introduced into a decantation funnel. The lower phase containing the pentafluoropropanol was separated and distilled in the presence of anhydrous H₂SO₄ (30 wt%) to give pure pentafluoropropanol containing 50 ppm of water (quantitative yield measured by gas chromatography (GC)). The yield in pentafluoropropanol was 99 %.

### Synthesis of difluoroethanol

3.4 g of 5 wt% Rh/C catalyst was introduced together with 37.4 g of water and 33.6 g of methyl difluoroacetate at room temperature into an autoclave reactor. The reactor was purged with helium. H₂ was bubbled in the solution via a dipping tube at a flow-rate of 8 l / h. The pressure was set to 40 bars and the reactor temperature was increased to 90°C. After 18 hours, the autoclave was cooled down to room temperature and vented. The collected liquid phase was filtered and distilled at atmospheric pressure. The yield in difluoroethanol was 74.4 %.

### Synthesis of perfluoropropionic acid

860 g CsF and 1246 g of diglyme (diethylene glycol dimethyl ether) were introduced into a 41 reactor. The reactor was then purged with N₂ and heated to 100°C. A cylinder containing a stoechiometric amount of CO₂ and tetrafluoroethylene at a total pressure of 25 bar was connected to the reactor. The gas mixture was then fed into the Cs/F / diglyme solution during 20 hours. A total amount of 550 g of tetrafluoroethylene and of 240 g of CO₂ was introduced during that period of time. The reactor was then cooled to room temperature. The collected liquid phase (2779 g) was mixed with 5 of diethyl ether to precipitate the cesium pentafluoroproprionate salt. 1480 g CF₃CF₂COOCs were collected after filtration and drying at 100°C during three days. The Cs salt was then hydrolyzed with concentrated H₂SO₄. 751 g perfluoropropionic acid (NMR purity = 98 %) were collected after decantation, corresponding to a total yield of 84 %.

## Claims

1. Process for the manufacture of fluorinated alcohols having the formula
CₙFₘH₂ₙ₊₁₋ₘCH₂OH (1)
wherein n is 1 or 2; and m is an integer from 1 to 5, but not larger than 2n+1;
by hydrogenating the corresponding fluorinated carboxylic acids and/or their derivates having the formula
CₙFₘH₂ₙ₊₁₋ₘCOR (2)
wherein n and m have the above meanings and R represents OH, Cl, Br, F, and OR', wherein R' is a hydrocarbon rest, in the presence of a catalyst and of water but excluding the hydrogenation of trifluoroacetic acid to form trifluoroethanol.

2. Process according to claim 1, wherein the concentration of water is from 10 to 70 % by weight, based upon the total weight of fluorinated carboxylic acids and/or their derivatives, water and catalyst.

3. Process according to claim 2, wherein the process is carried out at a temperature from 70 to 140°C.

4. Process according to any of claims 1 to 3, wherein the catalyst comprises at least one of the elements Ir, Ru, Rh on carbon.

5. Process according to any of claims 1 to 4, wherein the catalyst is used in an amount of 0.5 to 7 % by weight, based upon the total weight of fluorinated carboxylic acids and/or their derivatives, water and catalyst.

6. Process according to any of claims 1 to 5, wherein the hydrogenation is carried out under elevated hydrogen pressure.

7. Process according to claim 6, wherein the hydrogen pressure is from 1.5 to 70 bar.

8. Process according to any of claims 1 to 7, wherein the fluorinated alcohols to be produced are selected from the group consisting of pentafluoropropanol (CF₃CF₂CH₂OH), trifluoropropanol (CF₃CH₂CH₂OH) and difluoroethanol (CF₂HCH₂OH), and wherein these fluorinated alcohols are produced by hydrogenating the corresponding compounds selected from the group consisting of pentafluoropropionic acid (CF₃CF₂COOH), trifluoropropionic acid (CF₃CH₂COOH) and difluoroacetic acid (CF₂HCOOH).

9. Process according to any of claims 1 to 7, wherein difluoroethanol is produced by hydrogenating methyl or ethyl difluoroacetate.

10. Process according to claim 8, wherein pentafluoropropanol (CF₃CF₂CH₂OH) is produced by hydrogenating pentafluoropropionic acid (CF₃CF₂COOH).

11. Process according to claim 10, wherein pentafluoropropionic acid is produced by a method comprising the step :
reacting CF₂CF₂ with CO₂ and a source of fluoride ions in the presence of diethylene glycol dimethyl ether.

12. Process according to claim 11, wherein the source of fluoride ions is an alkali salt of hydrofluoric acid.
